# EUROPEAN PATENT SPECIFICATION

(11) **EP 2 007 292 B1**
(45) Date of publication and mention of the grant of the patent: **21.09.2016**
(21) Application number: 07760576.4
(22) Date of filing: 12.04.2007
(51) Int. Cl.: A61B 17/16

(54) **ACETABULAR TRIAL SYSTEM**
HÜFTPFANNENPRÜFSYSTEM
SYSTÈME D'ESSAI DE COTYLE PROTHÉTIQUE

(30) Priority: 12.04.2006 US 791590 P
(43) Date of publication of application: 31.12.2008
(73) Proprietor: Smith & Nephew, Inc., Memphis, Texxessee 38116 (US); Shotton, Vincent W., Magnolia, Texas 77354 (US)
(72) Inventor: SHOTTON, Vincent W., Texas 77354 (US)
(74) Representative: Smith & Nephew
(86) International application number: PCT/US2007/066545
(87) International publication number: WO 2007/121313

(56) References cited:
- WO-A-03/011116
- US-A1- 2004 117 029
- US-A1- 2005 049 713

## Description

### Background of the Invention

### 1. Field of the Invention

This invention relates generally to acetabular implants and, more particularly, to trial systems for the acetabulum.

### 2. Related Art

Many acetabular systems have a system and method of trialing the acetabulum to ensure that the components' sizing and location are satisfactory for example US 2004/117029. The method of trialing the acetabulum usually consists of provisionally implanting a trial acetabular shell and a trial liner. Some systems use different size trial shells and trial liners. Other systems include spacers to be placed between the trial liner and the trial shell to eliminate additional shells and liners within the trial system by building up the trial system using the spacers.

Some surgeons, in trialing the acetabular components of a hip implant, have used other methods and devices to size and locate the acetabular components. For example, some surgeons have reamed the acetabulum to a desired shape, and then used an additional, larger reamer to trial the acetabulum. The larger reamer may be one size larger than the reamer used on the acetabulum to check. The surgeon may check for circumferential coverage by inserting the larger reamer into the reamed acetabulum.

There remains a need in the art for an acetabular trial system that may properly size the acetabulum through the use of cutting tools.

In addition, the trial system may reduce both the number of tools in an operating room and operating room time by eliminating steps and/or making some steps easier.

### Summary of the Invention

In one aspect of the invention, an acetabular trial system comprises a first reamer, a second reamer and a liner. The first reamer has a first diameter and is configured to ream an acetabulum. The second reamer has a second diameter larger than the first diameter and is configured to ream the acetabulum. The liner is configured to mate to the second reamer such that when an acetabulum is reamed by the first reamer, the second reamer is sized as a trial shell component. The second reamer is capable of impaction into the acetabulum and reception of the liner such that the liner and the second reamer are configured to trial the acetabulum for size and orientation relative to a femoral component of a hip prosthesis.

A method which is not part of the invention for trialing an acetabulum is provided. The method comprises the steps of cutting an acetabulum using a first reamer. The method further provides impacting a second reamer having a diameter greater than the diameter of the first reamer into the cut acetabulum. Another step mates a liner to the second reamer such that the liner and the second reamer are configured to trial the acetabulum for size and orientation relative to a femoral component of a hip prosthesis.

An embodiment of the invention provides choosing the second reamer from a plurality of reamers having a plurality of diameters. Each of the plurality of reamers is configured to ream an acetabulum. The liner and each of the plurality of reamers are configured to trial the acetabulum for size and orientation relative to a femoral component of a hip prosthesis.

Another embodiment of the invention provides choosing a spacer configured to mate to the liner and the second reamer. The method includes mating an outer mating surface of the spacer to an inner surface of the second reamer. The method also includes mating an inner surface of the spacer to an outer surface of the liner. The spacer is configured to fill a radial distance between the inner surface of the second reamer and the outer surface of the liner.

Yet another embodiment of the invention provides mating a reamer adaptor to the first reamer. The reamer adaptor may attach to a drill configured to rotate the first reamer within an acetabulum.

In yet another aspect of the invention, an acetabular trial system comprises a plurality of reamers and a liner. The plurality of reamers having a plurality of diameters. Each of the plurality of reamers is configured to ream an acetabulum. The liner is configured to mate to at least one of the plurality of reamers such that when an acetabulum is reamed by one of the plurality of reamers, another of the plurality of reamers is sized as a trial shell component. The another of the plurality of reamers is capable of impaction into the acetabulum and reception of the liner such that the liner and the another of the plurality of reamers are configured to trial the acetabulum for size and orientation relative to a femoral component of a hip prosthesis.

The invention has several advantages over prior devices and techniques. First, the number of instruments in an operating room for an artificial hip surgery may be reduced. Second, the time in the operating room may be reduced because fewer steps may be necessary to complete the surgery and/or the steps may be easier to perform.

An embodiment of the invention provides an acetabular trial system wherein the second reamer comprises a plurality of reamers having a plurality of diameters. Each of the plurality of reamers is configured to ream an acetabulum and further capable of impaction into the acetabulum and reception of the liner such that the liner and each of the plurality of reamers are configured to trial the acetabulum for size and orientation relative to a femoral component of a hip prosthesis.

Another embodiment of the invention provides an acetabular trial system wherein the liner comprises a plurality of liners. Each of the plurality of liners is configured to mate to the second reamer. Each of the plurality of liners is further configured to receive a femoral head of a hip prosthesis.

Another embodiment of the invention further comprises a spacer configured to mate to the liner and the second reamer. The spacer comprises an outer mating surface, an inner mating surface, an outer mating surface, and a thickness. The outer mating surface is configured to mate the spacer to an inner surface of the second reamer. The inner mating surface is configured to mate the spacer to an outer surface of the liner. The thickness is configured to fill a radial distance between the inner surface of the second reamer and the outer surface of the liner.

Another embodiment of the invention provides an acetabular trial system wherein the liner further comprises a plurality of liners. At least one of the plurality of liners is configured to fill the radial distance between the second reamer and the liner.

Yet another embodiment of the invention provides an acetabular trial system further comprising a reamer adaptor configured to mate to the first reamer such that the reamer adaptor may attach to a drill configured to rotate the first reamer within an acetabulum.

Another embodiment of the invention provides an acetabular trial system wherein the reamer adaptor is further configured to attach to a connector on the first reamer. The connector is further configured to mate to the liner.

Another embodiment of the invention provides an acetabular trial system wherein the second reamer has a roughened outer surface.

Another embodiment of the invention provides an acetabular trial system wherein the second reamer further comprises a recess and the liner further comprises a tab. The tab is configured to mate to the recess.

Another embodiment of the invention provides an acetabular trial system wherein the recess comprises a plurality of recesses and the tab comprises a plurality of tabs.

Yet another embodiment of the invention provides an acetabular trial system wherein the second reamer further comprises a tab and the liner further comprises a recess. The tab is configured to mate to the recess.

Another embodiment of the invention provides an acetabular trial system wherein the recess comprises a plurality of recesses and the tab comprises a plurality of tabs.

Further features, aspects, and advantages of the present invention, as well as the structure and operation of various embodiments of the present invention, are described in detail below with reference to the accompanying drawings.

### Brief Description of the Drawings

The accompanying drawings, which are incorporated in and form a part of the specification, illustrate embodiments of the present invention and together with the description, serve to explain the principles of the invention. In the drawings:
Figure 1 is an exploded view of parts of a trial acetabular system for trialing an acetabulum.
Figure 2 is a view of the parts of Figure 1 assembled.
Figure 3 is an exploded view of parts of a trial acetabular system for reaming an acetabulum.
Figure 4 is a view of the parts of Figure 3 assembled.

### Detailed Description of the Embodiments

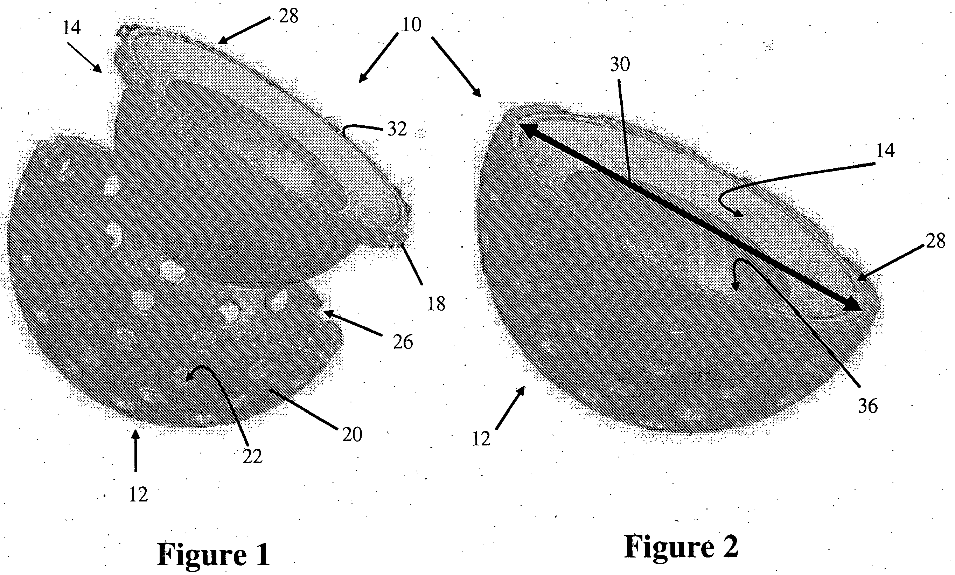

Referring to the accompanying drawings in which like reference numbers indicate like elements, Figure 1 is an exploded view of parts of a trial acetabular system 10 for trialing an acetabulum. The trial acetabular system 10 includes a reamer 12 and a trial liner 14. The liner 14 is seated within the reamer 12. The reamer 12 is placed within the reamed acetabulum (not shown). The reamer 12 serves as a trial acetabular shell component while the liner serves as a trial acetabular liner. Together, the system 10 is configured to orient the acetabular components of a hip implant relative to the femoral components of the hip implant.

The reamer 12 is sized with a larger diameter than the cutting tools used in cutting the acetabulum. The reamer 12 is impacted into the shaped acetabulum so that the reamer 12 is provisionally fixed to the acetabulum for measuring size and alignment before final fixation of the acetabular components of the implant.

The liner 14 is chosen so that the liner 14 is properly sized to receive the femoral plant of the implant the surgeon has determined be used in the surgery. Thus, because the femoral head size may be different for the same acetabular shell component, the reamer 12 may be configured to receive multiple trial liner 14 sizes. The liner 14 is further configured having a plurality of tabs 18 configured to retain the liner within the reamer 12, as described below.

the liner 14 may include a spacer 28 configured to mate to the reamer 12 at an inner diameter 30 (shown in Figure 2). The spacer has a thickness 32 which extends the liner 14 to the inner diameter 30 of the reamer 12. The spacer may be an integral component of the liner 14 or may be modularly attached to the liner 14 so that different spacers 28 of different thicknesses may be attached to the liner 14.

The acetabular reamer 12 includes a rough outer surface 20, cutting edges 22, and recesses 26. The recesses 26 are female connectors configured to receive the male connector tabs 18 of the liner 14. The liner 14 is mated to the reamer 12 by inserting the tabs 18 within the recesses 26. While this embodiment shows the female connector on the reamer 12 and the male connector on the liner 14, other embodiments may include the male component on the reamer 12 and the female connector on the liner. In addition, other connectors, such as, but not limited to, screws, clips, compression rings, interference fits or the like may be used to mate the liner 14 to the reamer 12.

Turning now to Figure 2, Figure 2 is a view of the parts of Figure 1 assembled. The liner 14 is mated to the reamer 12. The liner 14 has a femoral head receiving indent 36. The indent 36 may receive a femoral head component of a hip prosthesis. The size of the indent 36 may be based upon the size of the femoral head the surgeon has chosen to use in the procedure. The trial liner 14, then, may be modular with respect to the reamer 12 so that one reamer 12 may be used with a plurality of liners 14. In order to further minimize the number of parts in the system 10, the spacer 28 may also include a plurality of spacers such that a single liner 14 having an indent 36 may be mated to a plurality of reamers 12. As an example, assuming five reamer sizes and five indent sizes on five liners, then each reamer would need to be paired to five different liners for a total of 25 parts. But, if five spacers, each matching the inner diameter of a reamer on the spacers' outer surface, and all the spacers matching the outer diameter of the liners on the spacer's inner surface were used, then five spacers could be used individually to mate all of the reamers to all of the liners, thereby requiring 15 total parts. As the number of sizes of reamers and liners increases, the difference in the number of required parts increases dramatically.

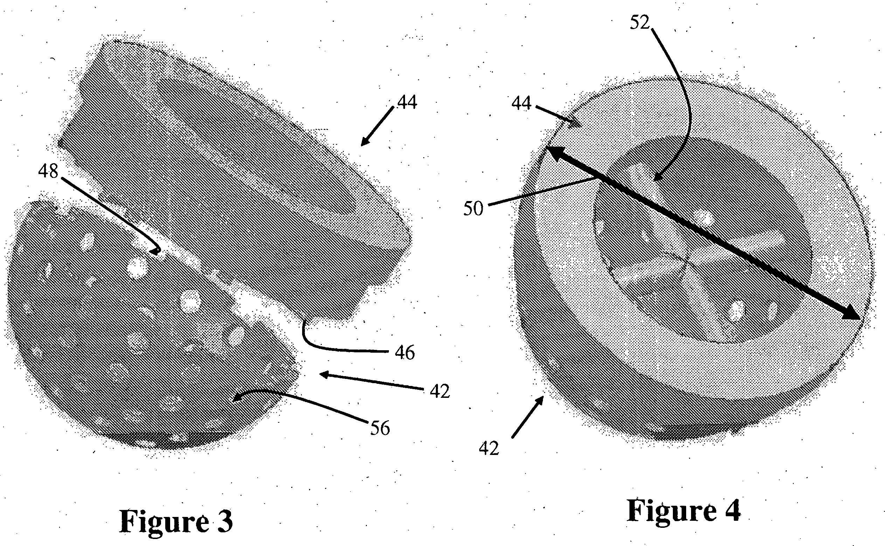

Turning now to Figures 3 and 4, Figure 3 is an exploded view of parts of a trial acetabular system for reaming an acetabulum. Figure 4 is a view of the parts of Figure 3 assembled. The parts include a reamer 42 and a reamer adaptor 44. The reamer adaptor 44 is configured having a plurality of tabs 46 configured to mate to a plurality of recesses 48 on the reamer 42. Together, the adaptor 44 and reamer 42 have an outer diameter 50. The outer diameter 50 is chosen by a surgeon so that the acetabular components may be effectively implanted into the acetabulum of a patient.

When the adaptor 44 is attached to the reamer 42, cross bar support members 52 of the adaptor 44 are configured to connect the reamer-adaptor assembly to a drill. While the cross bar members 52 are shown in this embodiment, other locking devices, mechanisms or methods may be used to connect the reamer to a drill. Cutting edges 56 of the reamer 42 are used to prepare the acetabulum to the desired diameter.

In this embodiment, the adaptor 44 attaches to the reamer 42 in the same recesses 48 where a liner would attach to the reamer 42. However, the adaptor 44 mates tangential to the radius while the liner of Figures 1 and 2 mates along a radius of the reamer 42. It may be beneficial to connect an adaptor tangential to the radius to minimize shear stresses transferred from the adaptor to the reamer, while it may be beneficial to seat the liner along the radius for clearance of the trial system.

After the reamer 42 has prepared the acetabulum, the second reamer 12, having a diameter greater than the diameter of the reamer used to ream the acetabulum, may be impacted into the acetabulum. By using a reamer as the trial shell, the roughened surface of the reamer, as well as the cutting surfaces, may provide a firmer grip of the trial to the bone. The liner may then be inserted into the impacted reamer, and the orientation and size of the hip prostheses may be trialed. Other liners may be used if the surgeon determines he would rather a different femoral head, thus requiring a different liner. The surgeon may then prepare the femur and perform a trial reduction of the hip prosthesis to ensure proper placement and correct sizing of the prosthesis. When the surgeon has finalized the placement and sizes of the hip prosthesis, then the trial liner and reamer are removed from the acetabulum and the prosthesis is implanted.

An acetabular trial system, then, comprises a first reamer, a second reamer and a liner. The first reamer has a first diameter and is configured to ream an acetabulum. The second reamer has a second diameter larger than the first diameter and is configured to ream the acetabulum. The liner is configured to mate to the second reamer such that when an acetabulum is reamed by the first reamer, the second reamer is sized as a trial shell component. The second reamer is capable of impaction into the acetabulum and reception of the liner such that the liner and the second reamer are configured to trial the acetabulum for size and orientation relative to femoral component of a hip prosthesis.

The method wich ist not part of the invention for trialing an acetabulum includes cutting an acetabulum using a first reamer. The method further provides impacting a second reamer having a diameter greater than the diameter of the first reamer into the cut acetabulum. Another step mates a liner to the second reamer such that the liner and the second reamer are configured to trial the acetabulum for size and orientation relative to a femoral component of a hip prosthesis.

While the trial reduction may be completed using only two reamers, it may be more common to provide a plurality of reamers to trial the acetabulum. The acetabular trial system then comprises a plurality of reamers and a liner. The plurality of reamers having a plurality of diameters. Each of the plurality of reamers is configured to ream an acetabulum. The liner is configured to mate to at least one of the plurality of reamers such that when an acetabulum is reamed by one of the plurality of reamers, another of the plurality of reamers is sized as a trial shell component. The another of the plurality of reamers is capable of impaction into the acetabulum and reception of the liner such that the liner and the another of the plurality of reamers are configured to trial the acetabulum for size and orientation relative to a femoral component of a hip prosthesis.

While a single liner may be used, another embodiment of the invention provides for a plurality of liners. Each of the plurality of liners is configured to mate to the second reamer. Each of the plurality of liners is further configured to receive a femoral head of a hip prosthesis. Together, a plurality of liners and reamers may be used to provide for more shell sizes and femoral head sizes in an implant. By using the plurality of reamers and liners, the reamers replace the need to provide trial shells in the trialing procedure.

In order to further reduce the number of components in the acetabular system, the system may include a spacer configured to mate to the liner and the second reamer. The spacer comprises an outer mating surface, an inner mating surface, an outer mating surface, and a thickness. The outer mating surface is configured to mate the spacer to an inner surface of the second reamer. The inner mating surface is configured to mate the spacer to an outer surface of the liner. The thickness is configured to fill a radial distance between the inner surface of the second reamer and the outer surface of the liner.

Another embodiment of the invention provides an acetabular trial system comprising a plurality of liners. At least one of the plurality of liners is configured to fill the radial distance between the second reamer and the liner. In such an embodiment, for one given femoral head size, the plurality of liners will have varying diameters such that each of the liners may fit into one of a plurality of different reamer diameters.

An acetabular trial system may further comprise a reamer adaptor configured to mate to the first reamer. The reamer adaptor may attach to a drill configured to rotate the first reamer within an acetabulum. The reamer, then, may be used in one instance to prepare an acetabulum and in another instance where the diameter of the acetabulum is desired to be smaller than the diameter of the first reamer, to be used as a trial insert.

Another embodiment provides an acetabular trial system where the reamer adaptor is further configured to attach to a connector on the first reamer. The connector is further configured to mate to the liner. Thus, the adaptor may be configured to mate to a plurality of reamers. Because each of the reamers may also be used with the adaptor to cut an acetabulum, the reamers may have a roughened outer surface.

The reamer of an acetabular trial system may include a recess and the liner may further comprise a tab. The tab is configured to mate to the recess so that the liner is mated to the reamer. Alternatively, the reamer may include a tab and the liner may include the recess. In addition, the recess may include a plurality of recesses and the tab may include a plurality of tabs. The tabs and/or recesses on the reamer may also be configured to mate the reamer adaptor to the reamer.

The embodiments were chosen and described in order to best explain the principles of the invention and its practical application to thereby enable others skilled in the art to best utilize the invention in various embodiments and with various modifications as are suited to the particular use contemplated.

In view of the foregoing, it will be seen that the several advantages of the invention are achieved and attained.

## Claims

1. An acetabular trial system (10),comprising:
a first reamer (42), having a first diameter (50) and configured to ream an acetabulum;
a second reamer (12) having a second diameter (30) larger than the first diameter and configured to ream the acetabulum; and
a liner (14) configured to mate to the second reamer such that when an acetabulum is reamed by the first reamer, the second reamer is sized as a trial shell component, the second reamer being capable of impaction into the acetabulum and reception of the liner such that the liner and the second reamer are configured to trial the acetabulum for size and orientation relative to a femoral component of a hip prosthesis.

2. The acetabular trial system of claim 1, wherein the second reamer comprises a plurality of reamers having a plurality of diameters, each of the plurality of reamers configured to ream an acetabulum and further capable of impaction into the acetabulum and reception of the liner such that the liner and each of the plurality of reamers are configured to trial the acetabulum for size and orientation relative to a femoral component of a hip prosthesis.

3. The acetabular trial system of claim 1 or 2, wherein the liner comprises a plurality of liners, each of the plurality of liners being configured to mate to the second reamer, each of the plurality of liners being further configured to receive a femoral head of a hip prosthesis.

4. The acetabular trial system of any of claims 1-3, further comprising a spacer (28) configured to mate to the liner and the second reamer, the spacer comprising:
an outer mating surface configured to mate the spacer to an inner surface of the second reamer,
an inner surface configured to mate the spacer to an outer surface of the liner, and
a thickness configured to fill a radial distance between the inner surface of the second reamer and the outer surface of the liner.

5. The acetabular trial system of any of claims 1-4, wherein the liner further comprises a plurality of liners, at least one of the plurality of liners being configured to fill a radial distance between the second reamer and the liner.

6. The acetabular trial system of any of claims 1-5, further comprising a reamer adaptor configured to mate to the first reamer such that the reamer adaptor may attach to a drill configured to rotate the first reamer within an acetabulum.

7. The acetabular trial system of any of claim 6, wherein the reamer adaptor is further configured to attach to a connector on the first reamer, the connector being further configured to mate to the liner.

8. The acetabular trial system of any of claims 1-7, wherein the second reamer has a roughened outer surface (20).

9. The acetabular trial system of any of claims 1-8, wherein the second reamer further comprises a recess and the liner further comprises a tab, the tab being configured to mate to the recess.

10. The acetabular trial system of claim 9, wherein the recess comprises a plurality of recesses (48) and the tab comprises a plurality of tabs (46).

11. The acetabular trial system of any of claims 1-8, wherein the second reamer further comprises a tab and the liner further comprises a recess, the tab being configured to mate to the recess.

12. The acetabular trial system of claim 11, wherein the recess comprises a plurality of recesses and the tab comprises a plurality of tabs.

## Patentansprüche

1. Ein Hüftpfannentestsystem (10), das Folgendes beinhaltet:
eine erste Fräsvorrichtung (42), die einen ersten Durchmesser (50) aufweist und zum Fräsen einer Hüftpfanne konfiguriert ist;
eine zweite Fräsvorrichtung (12), die einen zweiten Durchmesser (30) aufweist, der größer ist als der erste Durchmesser, und zum Fräsen der Hüftpfanne konfiguriert ist; und
einen Einsatz (14), der konfiguriert ist, um an die zweite Fräsvorrichtung zu passen, so dass, wenn eine Hüftpfanne von der ersten Fräsvorrichtung gefräst wird, die zweite Fräsvorrichtung als eine Testschalenkomponente bemessen ist, wobei die zweite Fräsvorrichtung in der Lage ist, sich in die Hüftpfanne einzuklemmen und den Einsatz aufzunehmen, so dass der Einsatz und die zweite Fräsvorrichtung konfiguriert sind, um die Hüftpfanne bezüglich der Größe und Ausrichtung relativ zu einer Femurkomponente einer Hüftprothese zu testen.

2. Hüftpfannentestsystem gemäß Anspruch 1, wobei die zweite Fräsvorrichtung eine Vielzahl von Fräsvorrichtungen mit einer Vielzahl von Durchmessern beinhaltet, wobei jede der Vielzahl von Fräsvorrichtungen zum Fräsen einer Hüftpfanne konfiguriert ist und ferner in der Lage ist, sich in die Hüftpfanne einzuklemmen und den Einsatz aufzunehmen, so dass der Einsatz und jede der Vielzahl von Fräsvorrichtungen konfiguriert sind, um die Hüftpfanne bezüglich der Größe und Ausrichtung relativ zu einer Femurkomponente einer Hüftprothese zu testen.

3. Hüftpfannentestsystem gemäß Anspruch 1 oder 2, wobei der Einsatz eine Vielzahl von Einsätzen beinhaltet, wobei jeder der Vielzahl von Einsätzen konfiguriert ist, um an die zweite Fräsvorrichtung zu passen, wobei jeder der Vielzahl von Einsätzen ferner konfiguriert ist, um einen Femurkopf einer Hüftprothese aufzunehmen.

4. Hüftpfannentestsystem gemäß einem der Ansprüche 1-3, ferner beinhaltend einen Abstandshalter (28), der konfiguriert ist, um an den Einsatz und die zweite Fräsvorrichtung zu passen, wobei der Abstandshalter Folgendes beinhaltet:
eine Außenpassfläche, die konfiguriert ist, um den Abstandshalter an eine Innenfläche der zweiten Fräsvorrichtung zu passen,
eine Innenfläche, die konfiguriert ist, um den Abstandshalter an eine Außenfläche des Einsatzes zu passen, und
eine Dicke, die konfiguriert ist, um einen radialen Abstand zwischen der Innenfläche der zweiten Fräsvorrichtung und der Außenfläche des Einsatzes zu füllen.

5. Hüftpfannentestsystem gemäß einem der Ansprüche 1-4, wobei der Einsatz ferner eine Vielzahl von Einsätzen beinhaltet, wobei mindestens einer der Vielzahl von Einsätzen konfiguriert ist, um einen radialen Abstand zwischen der zweiten Fräsvorrichtung und dem Einsatz zu füllen.

6. Hüftpfannentestsystem gemäß einem der Ansprüche 1-5, ferner beinhaltend ein Fräsvorrichtungszwischenstück, das konfiguriert ist, um an die erste Fräsvorrichtung zu passen, so dass das Fräsvorrichtungszwischenstück an einer Bohrvorrichtung angebracht werden kann, die konfiguriert ist, um die erste Fräsvorrichtung innerhalb einer Hüftpfanne zu drehen.

7. Hüftpfannentestsystem gemäß einem der Ansprüche 6, wobei das Fräsvorrichtungszwischenstück ferner konfiguriert ist, um an ein Verbindungsstück auf der ersten Fräsvorrichtung angebracht zu werden, wobei das Verbindungsstück ferner konfiguriert ist, um an den Einsatz zu passen.

8. Hüftpfannentestsystem gemäß einem der Ansprüche 1-7, wobei die zweite Fräsvorrichtung eine geraute Außenfläche (20) aufweist.

9. Hüftpfannentestsystem gemäß einem der Ansprüche 1-8, wobei die zweite Fräsvorrichtung ferner eine Aussparung beinhaltet und der Einsatz ferner einen Vorsprung beinhaltet, wobei der Vorsprung konfiguriert ist, um in die Aussparung zu passen.

10. Hüftpfannentestsystem gemäß Anspruch 9, wobei die Aussparung eine Vielzahl von Aussparungen (49) beinhaltet und der Vorsprung eine Vielzahl von Vorsprüngen (46) beinhaltet.

11. Hüftpfannentestsystem gemäß einem der Ansprüche 1-8, wobei die zweite Fräsvorrichtung ferner einen Vorsprung beinhaltet und der Einsatz ferner eine Aussparung beinhaltet, wobei der Vorsprung konfiguriert ist, um in die Aussparung zu passen.

12. Hüftpfannentestsystem gemäß Anspruch 11, wobei die Aussparung eine Vielzahl von Aussparungen beinhaltet und der Vorsprung eine Vielzahl von Vorsprüngen beinhaltet.

## Revendications

1. Un système d'essai acétabulaire (10), comprenant :
un premier alésoir (42), ayant un premier diamètre (50) et configuré pour aléser un acétabulum ;
un deuxième alésoir (12) ayant un deuxième diamètre (30) plus grand que le premier diamètre et configuré pour aléser l'acétabulum ; et
un revêtement intérieur (14) configuré pour s'accoupler au deuxième alésoir de telle sorte que lorsqu'un acétabulum est alésé par le premier alésoir, le deuxième alésoir soit dimensionné comme un composant d'enveloppe d'essai, le deuxième alésoir étant capable d'une impaction dans l'acétabulum et d'une réception du revêtement intérieur de telle sorte que le revêtement intérieur et le deuxième alésoir soient configurés pour tester l'acétabulum pour la dimension et l'orientation relativement à un composant fémoral d'une prothèse de hanche.

2. Le système d'essai acétabulaire de la revendication 1, dans lequel le deuxième alésoir comprend une pluralité d'alésoirs ayant une pluralité de diamètres, chaque alésoir de la pluralité d'alésoirs étant configuré pour aléser un acétabulum et étant en outre capable d'une impaction dans l'acétabulum et d'une réception du revêtement intérieur de telle sorte que le revêtement intérieur et chaque alésoir de la pluralité d'alésoirs soient configurés pour tester l'acétabulum pour la dimension et l'orientation relativement à un composant fémoral d'une prothèse de hanche.

3. Le système d'essai acétabulaire de la revendication 1 ou de la revendication 2, dans lequel le revêtement intérieur comprend une pluralité de revêtements intérieurs, chaque revêtement intérieur de la pluralité de revêtements intérieurs étant configuré pour s'accoupler au deuxième alésoir, chaque revêtement intérieur de la pluralité de revêtements intérieurs étant en outre configuré pour recevoir une tête fémorale d'une prothèse de hanche.

4. Le système d'essai acétabulaire de n'importe lesquelles des revendications 1 à 3, comprenant en outre une pièce d'espacement (28) configurée pour s'accoupler au revêtement intérieur et au deuxième alésoir, la pièce d'espacement comprenant :
une surface d'accouplement externe configurée pour accoupler la pièce d'espacement à une surface interne du deuxième alésoir,
une surface interne configurée pour accoupler la pièce d'espacement à une surface externe du revêtement intérieur, et
une épaisseur configurée pour remplir une distance radiale entre la surface interne du deuxième alésoir et la surface externe du revêtement intérieur.

5. Le système d'essai acétabulaire de n'importe lesquelles des revendications 1 à 4, dans lequel le revêtement intérieur comprend en outre une pluralité de revêtements intérieurs, au moins un revêtement intérieur de la pluralité de revêtements intérieurs étant configuré pour remplir une distance radiale entre le deuxième alésoir et le revêtement intérieur.

6. Le système d'essai acétabulaire de n'importe lesquelles des revendications 1 à 5, comprenant en outre un adaptateur d'alésoir configuré pour s'accoupler au premier alésoir de telle sorte que l'adaptateur d'alésoir puisse s'attacher à un foret configuré pour faire tourner le premier alésoir au sein d'un acétabulum.

7. Le système d'essai acétabulaire de n'importe lesquelles de la revendication 6, dans lequel l'adaptateur d'alésoir est en outre configuré pour s'attacher à un élément de raccordement sur le premier alésoir, l'élément de raccordement étant en outre configuré pour s'accoupler au revêtement intérieur.

8. Le système d'essai acétabulaire de n'importe lesquelles des revendications 1 à 7, dans lequel le deuxième alésoir a une surface externe rugueuse (20).

9. Le système d'essai acétabulaire de n'importe lesquelles des revendications 1 à 8, dans lequel le deuxième alésoir comprend en outre un renfoncement et le revêtement intérieur comprend en outre une patte, la patte étant configurée pour s'accoupler au renfoncement.

10. Le système d'essai acétabulaire de la revendication 9, dans lequel le renfoncement comprend une pluralité de renfoncements (49) et la patte comprend une pluralité de pattes (46).

11. Le système d'essai acétabulaire de n'importe lesquelles des revendications 1 à 8, dans lequel le deuxième alésoir comprend en outre une patte et le revêtement intérieur comprend en outre un renfoncement, la patte étant configurée pour s'accoupler au renfoncement.

12. Le système d'essai acétabulaire de la revendication 11, dans lequel le renfoncement comprend une pluralité de renfoncements et la patte comprend une pluralité de pattes. MURG * 180964
